# DEMANDE DE BREVET EUROPEEN

(11) **EP 1 462 094 A1**
(43) Date de publication de la demande: **29.09.2004**
(21) Numéro de dépôt: 04290412.8
(22) Date de dépôt: 16.02.2004
(51) Int. Cl.: A61K 7/48

(54) **Composition à usage cosmétique ou dermatologique contenant un polymère bloc**

(30) Priorité: 19.03.2003 FR 0303356
(71) Demandeur: L'OREAL, 75008 Paris (FR)
(72) Inventeur: L'Alloret, Florence, 75013 Paris (FR)
(74) Mandataire: Rasson, Catherine

(57) **Abrégé**

La présente demande concerne une composition cosmétique et/ou dermatologique comprenant au moins une phase aqueuse comportant au moins un polymère hydrosoluble ou hydrodispersible, choisi parmi les polymères triblocs ou diblocs comportant un bloc hydrophobe ayant une température de transition vitreuse supérieure ou égale à 70°C.

La composition présente l'avantage d'être bien stable, même lorsqu'elle se présente sous forme d'une émulsion contenant des particules solides tels que le dioxyde de titane.

L'invention a aussi pour objet l'utilisation de ladite composition, notamment dans le domaine cosmétique pour le soin, le nettoyage, la protection et/ou le maquillage de la peau, des fibres kératiniques et/ou des muqueuses.

## Description

La présente demande se rapporte à une composition cosmétique et/ou dermatologique comprenant au moins une phase aqueuse comportant au moins un polymère hydrosoluble ou hydrodispersible, choisi parmi les polymères triblocs ou diblocs comportant un bloc hydrophobe ayant une température de transition vitreuse supérieure ou égale à 70°C, et à ses utilisations dans le domaine cosmétique ou dermatologique, notamment pour le soin, le nettoyage, la protection et/ou le maquillage des matières kératiniques (peau, muqueuses, fibres kératiniques telles que cheveux et cils).

Une grande majorité de compositions cosmétiques comprennent des épaississants ou gélifiants utilisés pour ajuster les propriétés de texture en fonction de l'application visée. Les supports galéniques les plus représentatifs sont :
- les lotions ou sérums aqueux ne comprenant pas ou peu de phase grasse,
- les émulsions qui sont des dispersions de deux liquides non miscibles tels que l'eau et l'huile. On distingue les émulsions directes huile-dans-eau (H/E) comprenant une phase huileuse dispersée dans une phase aqueuse, des émulsions inverses eau-dans-huile (E/H) qui sont des dispersions d'une phase aqueuse dans une phase huileuse. Il existe également des émulsions multiples obtenues après dispersion d'une émulsion inverse dans une phase aqueuse (E/H/E) ou après dispersion d'une émulsion directe dans une phase huileuse (H/E/H). On entend ici par « émulsions » aussi bien les dispersions obtenues en absence de tensioactifs émulsionnants que les émulsions obtenues en présence de tensioactifs émulsionnants.

La nature des composés utilisés pour gélifier la phase aqueuse et leur teneur dans la composition sont choisies en fonction du type de texture désiré. Les principaux agents épaississants ou gélifiants hydrophiles pouvant être utilisés dans le domaine cosmétique sont les suivants :
- les gélifiants polymériques réticulés tels que les Carbopols (Goodrich) ou les polymères d'acide 2-acrylamido 2-méthylpropane sulfonique réticulés et au moins partiellement neutralisés comme par exemple le produit commercialisé sous la dénomination Hostacerin AMPS par la société Clariant. Ces agents gélifiants ne permettent pas de stabiliser seuls une phase huileuse à une teneur supérieure à 10% en poids, "seuls" signifiant en l'absence de tensioactifs habituellement utilisés dans les émulsions ou en l'absence de tout autre agent stabilisant, ce qui limite le type de formulations sans tensioactif pouvant être préparées, ces compositions sans tensioactif classique étant pourtant recherchées pour leur bonne innocuité vis à vis de la peau ;
- les polymères naturels tels que les gommes de xanthane et de guar ou encore les dérivés cellulosiques, les amidons et les alginates. Ces composés sont en général utilisés en association avec les gélifiants polymériques réticulés car leur pouvoir épaississant n'est pas suffisamment important pour qu'ils puissent être utilisés comme seuls gélifiants.

La stabilité des compositions cosmétiques en fonction du temps et sur une large gamme de température (5°C - 50°C) est un paramètre crucial à prendre en compte lors de l'élaboration d'une nouvelle composition. La présence d'une phase dispersée liquide (émulsion) ou solide (suspension) peut induire des phénomènes de déstabilisation tels que la sédimentation (dépôt de solide), le crémage (séparation partielle des phases aqueuse et huileuse), ou encore l'agrégation et la coalescence, cette déstabilisation pouvant se traduire à l'échelle microscopique et/ou macroscopique. Une solution pour limiter ces phénomènes de déstabilisation consiste à augmenter la viscosité de la phase aqueuse (phase continue des H/E) à l'aide des agents épaississants / gélifiants précédemment cités, ce qui implique une concentration en polymère élevée dans la composition, mais cette dernière présente alors un toucher filmogène et collant après application sur la peau. Par ailleurs, pour des viscosités moins importantes, l'introduction de ces agents épaississants / gélifiants peut ne pas être suffisante pour éviter la déstabilisation des compositions.

En outre, les compositions cosmétiques peuvent également comprendre toutes sortes d'additifs et notamment des particules solides telles que des pigments, des filtres UV inorganiques ou des charges. Ces particules solides peuvent aussi déstabiliser les émulsions et entraîner des phénomènes de sédimentation au cours du temps.

Le document WO-A-01/16187 (Rhodia) décrit des copolymères blocs, comprenant au moins un bloc de nature hydrosoluble comprenant aussi des motifs hydrophobes et au moins un bloc de nature majoritairement hydrophobe, qui, en solution dans l'eau, forment un gel viscoélastique. Toutefois, comme indiqué ci-après, les polymères utilisés selon la présente invention ne sont pas décrits dans ce document et ils permettent d'obtenir des résultats qui ne peuvent pas être obtenus avec les polymères décrits dans ce document.

Les documents EP-A-1,279,398 et EP-A-1,287,395 décrivent des compositions cosmétiques contenant des polymères diblocs et triblocs permettant d'obtenir des compositions stables dans une large gamme de textures. Toutefois, il a été observé que les polymères spécifiquement décrits dans ces documents ne permettent pas d'obtenir des compositions bien stables quand elles contiennent des particules solides telles que citées ci-dessus.

Il subsiste donc le besoin de disposer d'un composé permettant de gélifier la phase aqueuse d'une émulsion afin d'obtenir une large gamme de textures, tout en étant facile à disperser dans l'eau, dont le pouvoir épaississant/gélifiant soit reproductible et qui, par ailleurs, de manière préférée, présente à la fois des propriétés gélifiantes et émulsionnantes, ce composé permettant en outre d'obtenir une composition présentant de bonnes propriétés cosmétiques et ayant une bonne stabilité, quelle que soit la teneur en huiles et/ou même en présence de particules solides.

La demanderesse a découvert de façon inattendue que des polymères blocs particuliers permettaient d'atteindre le but de l'invention. Ces polymères blocs sont des polymères hydrosolubles ou hydrodispersibles de structure triblocs B-A-B, ou de structure diblocs A-B, où A est un bloc polymérique hydrosoluble ionique et B un bloc polymérique hydrophobe, où la quantité de bloc polymérique A est égale ou supérieure à 50 % du poids total du polymère B-A-B, et égale ou supérieure à 60 % du poids total du polymère A-B, et où le bloc B hydrophobe a une température de transition vitreuse est supérieure ou égale à 70°C. Ces polymères présentent des propriétés de structuration de phase aqueuse, à des concentrations massiques inférieures à 20%, et ils permettent d'obtenir non seulement des compositions cosmétiques caractérisées par une excellente stabilité, même pour des émulsions comprenant plus de 10% de phase huileuse, ce qui permet d'avoir une large gamme de textures ayant de bonnes propriétés cosmétiques, mais encore des compositions contenant des particules solides qui soient stables dans le temps. La solubilisation ou dispersion de ces polymères dans l'eau est facile et les propriétés de gélification obtenues sont reproductibles en fonction des différents lots.

Ainsi, la présente invention a pour objet une composition pour application topique, caractérisée en ce qu'elle comprend au moins une phase aqueuse comportant au moins un polymère hydrosoluble ou hydrodispersible, choisi parmi
(1) les polymères de structure triblocs B-A-B où A est un bloc polymérique hydrosoluble ionique et B un bloc polymérique hydrophobe, où la quantité de bloc polymérique A est égale ou supérieure à 50 % du poids total du polymère triblocs B-A-B, et où le bloc B hydrophobe a une température de transition vitreuse supérieure ou égale à 70°C, et/ou (2) les polymères de structure diblocs A-B, où A est un bloc polymérique hydrosoluble ionique et B un bloc polymérique hydrophobe, où la quantité de bloc polymérique A est égale ou supérieure à 60 % du poids total du polymère diblocs A-B et où le bloc B hydrophobe a une température de transition vitreuse supérieure ou égale à 70°C.

On entend ici par « application topique » une application externe sur les matières kératiniques, que sont notamment la peau, le cuir chevelu, les cils, les sourcils, les ongles, les muqueuses et les cheveux.

La composition selon l'invention étant destinée à une application topique, elle contient un milieu physiologiquement acceptable, c'est-à-dire compatible avec toutes les matières kératiniques telles que la peau, les ongles, les muqueuses et les cheveux ou toute autre zone cutanée du corps. Les compositions selon l'invention peuvent constituer notamment des compositions cosmétiques ou dermatologiques.

Par « hydrosoluble ou hydrodispersible », on entend dans la présente demande, des polymères qui, introduits dans une phase aqueuse à 25°C, à une concentration en poids égale à 1%, permettent l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 70%, de préférence d'au moins 80%.

Par « bloc polymérique », on entend dans la présente demande un polymère bloc dont la masse molaire est supérieure à 400 g/mole, et de préférence supérieure à 800 g/mole.

Par « bloc hydrophobe », on entend dans la présente demande, un polymère bloc qui, introduit dans un solvant hydrocarboné à 25°C, à une concentration en poids égale à 1%, permet l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 70%, de préférence d'au moins 80%. Le solvant hydrocarboné utilisé ici a une constante diélectrique mesurée à 25°C, inférieure à 50 ; ce solvant peut être notamment choisi parmi les alcanes tels que le cyclohexane (constante diélectrique : 2,02) ; les solvants aromatiques tels que l'éthylbenzène (constante diélectrique : 2,4) ; les cétones telles que la cyclohexanone (constante diélectrique : 18,3) ; les éthers tels que le diéthyléther (constante diélectrique = 4,4) ; les alcools tels que le cyclohexanol (constante diélectrique: 15,0); les solvants chlorés hydrocarbonés tels que le dichlorométhane (constante diélectrique : 9,08) ; les amides telles que la diméthylformamide ; et les esters tels que l'acétate d'éthyle (constante diélectrique : 6,02).

Par « température de transition vitreuse », on entend la température au delà de laquelle un polymère amorphe devient plus mou, cette température étant inférieure à la température de fusion (Polymer Handbook, 3^{rd} edition, J. Brandrup, E.H. Immergut, J. Wiley & Sons, 1989). Le phénomène de transition vitreuse se traduit par des changements de propriétés du polymère telles que la chaleur spécifique. La température de transition vitreuse peut donc être évaluée par analyse thermique différentielle, selon la norme ISO 11357-2.

La température de transition vitreuse est dépendante de la masse molaire. Ainsi, la masse molaire d'un polystyrène dont la température de transition vitreuse est égale à 70°C, est de 3340 g/mole [Valeur calculée d'après la relation suivante donnée dans le "Polymer Handbook", Third Edition, édité par J. Brandrup, E.H. Immergut, 1989, page V/82 : Tg (K) = 373 - (100000/M)].

Les polymères utilisés selon l'invention permettent d'obtenir une stabilisation satisfaisante des phases aqueuses des compositions pour application topique, notamment cosmétiques ou dermatologiques, contenant des particules solides.

L'invention a aussi pour objet l'utilisation d'au moins un polymère hydrosoluble ou hydrodispersible, choisi parmi (1) les polymères de structure triblocs B-A-B où A est un bloc polymérique hydrosoluble ionique et B un bloc polymérique hydrophobe, où la quantité de bloc polymérique A est égale ou supérieure à 50 % du poids total du polymère triblocs B-A-B, et où le bloc B hydrophobe a une température de transition vitreuse supérieure ou égale à 70°C, et/ou (2) les polymères de structure diblocs A-B, où A est un bloc polymérique hydrosoluble ionique et B un bloc polymérique hydrophobe, où la quantité de bloc polymérique A est égale ou supérieure à 60 % du poids total du polymère diblocs A-B et où le bloc B hydrophobe a une température de transition vitreuse supérieure ou égale à 70°C, pour stabiliser une composition cosmétique et/ou dermatologique comprenant au moins une phase aqueuse et des particules solides.

Par ailleurs, les polymères utilisés dans la composition selon l'invention ont des propriétés émulsionnantes et peuvent également permettre la préparation d'émulsion exempte de tensioactif émulsionnant, c'est-à-dire ne contenant pas ou contenant de faibles teneurs en tensioactif émulsionnant (de 0 à environ 1 % en poids par rapport au poids total de la composition et de préférence moins de 0,5 % en poids), notamment d'émulsions H/E. On entend dans la présente demande par « tensioactif émulsionnant » les tensioactifs monomères ayant des propriétés émulsionnantes.

### Polymère triblocs ou diblocs

Les polymères utilisés selon la présente invention se différencient de ceux décrits dans le document WO-A-01/16187 par le fait qu'ils comportent une proportion bien déterminée de bloc polymérique A (pour le polymère triblocs : égale ou supérieure à 50 % du poids total du polymère triblocs, et pour le polymère diblocs : égale ou supérieure à 60 % dans le polymère dibloc, ce qui correspond à des proportions en bloc hydrophobe respectivement inférieures à 50 % et 40 %), alors que, dans ce document, la proportion massique entre les blocs majoritairement hydrophile et hydrophobe est comprise entre 95/5 et 20/80.

Dans les exemples 1 à 6 de ce document concernant les polymères diblocs, la proportion en bloc hydrophobe est supérieure à 40 %. Par ailleurs, les polymères diblocs et triblocs ayant une proportion massique en bloc hydrophobe, inférieure à 50 % pour les polymères triblocs ou inférieure à 40 % pour les polymères diblocs, donnés en exemple dans ce document (exemples 7, 10 et 11), présentent une température de transition vitreuse bien inférieure à 70°C, comme le montre le tableau ci-dessous.

| Exemple | Bloc hydrophobe (bloc B) | | | |
|---|---|---|---|---|
| | Nature | Masse molaire (g/mole) du bloc | Proportion massique dans le copolymère (%) | Tg (°C) |
| 1 | Styrène Acide méthacrylique Méthacrylate de 2-hydroxyéthyle | 5900 | 45,7 (bloc A = 54,3%) | Non calculée |
| 2 | Styrène Acide méthacrylique | 6500 | 48,1 (bloc A = 50,9%) | Non calculée |
| 3 | Styrène Méthacrylate de 2-hydroxyéthyle | 6400 | 50,6 (bloc A = 49,4%) | Non calculée |
| 4 | Styrène Acide méthacrylique | 6300 | 47,4 (bloc A = 52,6%) | Non calculée |
| 5 | Styrène Acide méthacrylique Méthacrylate de 2-hydroxyéthyle | 6900 | 51,3 (bloc A = 48,7%) | Non calculée |
| 6 | Styrène Acide acrylique | 7400 | 53,5 (bloc A = 46,5%) | Non calculée |
| 7 | Styrène | 2600 | 15,4 (bloc A = 84,6%) | 62 |
| 10 | Styrène 98% (en poids) Acide méthacrylique 2% | 2000 | 9,8 (bloc A = 90,2%) | 50 |
| 11 | Styrène 98% (en poids) | 2000 | 9,8 | 50 |
| | Acide méthacrylique 2% en poids | 500 | 2,5 | < 50 |

Ainsi, les exemples décrits dans ce document sont différents des polymères utilisés selon l'invention puisque :
- soit la proportion massique du bloc hydrophile est inférieure à 60% (exemples 1 à 6),
- soit le bloc hydrophobe possède une température de transition vitreuse inférieure à 70°C (exemples 7, 10 et 11).

D'autre part, les polymères du document WO-A-01/16187 sont décrits pour leurs propriétés gélifiantes de l'eau mais il n'est pas décrit qu'ils puissent avoir un pouvoir émulsionnant.

La proportion massique du bloc hydrophile ionique A dans les polymères diblocs A-B de l'invention est égale ou supérieure à 60 %, de préférence supérieure à 60 % et mieux supérieure à 70% par rapport au poids total des blocs A et B.

La proportion massique du bloc hydrophile ionique A dans les polymères triblocs B-A-B de l'invention est égale ou supérieure à 50 %, de préférence supérieure à 50 % et mieux de préférence supérieure à 60 % par rapport au poids total des blocs A et B.

Le bloc hydrosoluble ionique A des polymères de l'invention est obtenu à partir d'un ou plusieurs monomères hydrosolubles (la) ou les sels de ceux-ci, comme par exemple :
- l'acide (méth)acrylique,
- l'acide styrène sulfonique,
- l'acide vinylsulfonique et l'acide (méth)allylsulfonique,
- l'acide vinyl phosphonique,
- l'acide maléique,
- l'acide itaconique,
- l'acide crotonique,
- le chlorure de diméthyldiallyl ammonium;
- le chlorure de méthylvinylimidazolium,
- les carboxybétaïnes ou sulfobétaïnes éthyléniques, obtenues par exemple par quaternisation de monomères à insaturation éthylénique comportant une fonction amine, par des sels de sodium d'acide carboxylique à halogène mobile (ex : chloroacétate) ou par des sulfones cycliques (ex : propane sulfone).
- les monomères vinyliques hydrosolubles de formule (I) suivante : 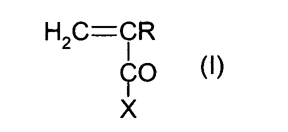
dans laquelle :
- R est choisi parmi H, -CH₃, -C₂H₅ ou -C₃H₇
- X est choisi parmi :
   - les oxydes d'alkyle de type -OR₁ où R₁ est un radical hydrocarboné, linéaire ou ramifié, saturé ou insaturé, ayant de 1 à 6 atomes de carbone, substitué par au moins un groupement sulfonique (-SO₃⁻) et/ou sulfate (-SO₄⁻) et/ou phosphate (-PO₄H₂⁻) et/ou un ammonium (-N⁺R₂R₃R₄), les radicaux R₂, R₃ et R₄ étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R₂ + R₃ + R₄ ne dépasse pas 6. Le radical R₁ est éventuellement substitué par un atome d'halogène (iode, brome, chlore, fluor); hydroxy (-OH); éther (-O-), amine primaire (-NH₂); amine secondaire (-NHR') ; amine tertiaire (-NR'R"), les radicaux R' et R", étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R' + R" ne dépasse pas 6. Comme monomères vinyliques comportant des groupes ester (X=OR₁), on peut citer par exemple le méthacrylate de diméthylaminoéthyl quaternisé (MADAME).
   - les groupements -NH₂, NHR₅ et -NR₅R₆ dans lesquels R₅ et R₆ sont indépendamment l'un de l'autre un radical hydrocarboné, linéaire ou ramifié, saturé ou insaturé ayant 1 à 6 atomes de carbone, sous réserve que le nombre total d'atomes de carbone de R₅ + R₆ ne dépasse pas 6, lesdits radicaux R₅ et/ou R₆ étant substitués par au moins un groupement sulfonique (-SO₃⁻) et/ou sulfate (-SO₄⁻) et/ou phosphate (-PO₄H₂⁻) et/ou amine quaternaire (-N⁺R₇R₈R₉), les radicaux R₇, R₈ et R₉ étant, indépendamment l'un de l'autre, un radical hydrocarboné, linéaire ou ramifié, saturé ou insaturé ayant 1 à 6 atomes de carbone, sous réserve que la somme des atomes de carbone de R₅ + R₆ + R₇ + R₈ + R₉ ne dépasse pas 6. Les radicaux R₅ et/ou R₆ peuvent éventuellement être substitués par un atome d'halogène (iode, brome, chlore, fluor); un groupement hydroxy (-OH); éther (-O-), amine primaire (-NH₂); amine secondaire (-NHR') ; amine tertiaire (-NR'R"), les radicaux R" et R", étant, indépendamment l'un de l'autre, un radical hydrocarboné, linéaire ou ramifié, saturé ou insaturé ayant 1 à 6 atomes de carbone, sous réserve que la somme des atomes de carbone de R₅ + R₆ + R' + R" ne dépasse pas 6. Comme monomères vinyliques comportant des groupes amide, on peut citer par exemple l'acide 2-acrylamido-2-méthylpropane sulfonique (AMPS), le chlorure de (méth)acrylamidopropyltriméthylammonium (APTAC et MAPTAC).

Dans la formule (I), les substituants R et X sont tels que le monomère de formule (I) est hydrosoluble.

A côté des monomères hydrosolubles (la) indiqués ci-dessus toujours présents dans le bloc hydrosoluble ionique A, celui-ci peut également contenir éventuellement un ou plusieurs monomères hydrosolubles neutres (Ib), comme par exemple :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyle cyclique ayant de 4 à 9 atomes de carbone, tels que la N-vinylpyrrolidone, la N-butyrolactame et la N-vinylcaprolactame,
- l'alcool vinylique de formule CH₂=CHOH,
- les monomères vinyliques hydrosolubles de formule (II) suivante : 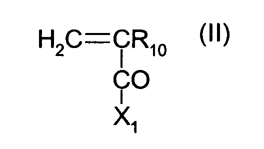
dans laquelle :
- R₁₀ est choisi parmi H, -CH₃, -C₂H₅ ou -C₃H₇
- X₁ est choisi parmi :

- les oxydes d'alkyle de type -OR₁₁ où R₁₁ est un radical hydrocarboné, linéaire ou ramifié, saturé ou insaturé, ayant de 1 à 6 carbones, éventuellement substitué par un atome d'halogène (iode, brome, chlore, fluor) ; un groupement hydroxy (-OH); éther (-O-), amine primaire (-NH₂); amine secondaire (-NHR') ; amine tertiaire (-NR'R"), les radicaux R' et R" étant, indépendamment l'un de l'autre, un radical hydrocarboné, linéaire ou ramifié, saturé ou insaturé ayant 1 à 6 atomes de carbone, sous réserve que la somme des atomes de carbone de R₁₁ + R' + R" ne dépasse pas 6. On peut citer par exemple comme monomères de formule (II) où X₂ est un radical - OR₁₁, le (méth)acrylate de glycidyle, le méthacrylate d'hydroxyéthyle, et les (méth)acrylates d'éthylène glycol, de diéthylèneglycol ou de polyalkylèneglycol.
- les groupements -NH₂, -NHR₁₂ et -NR₁₂R₁₃ dans lesquels R₁₂ et R₁₃ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₂ et R₁₃ ne dépasse pas 6, les radicaux R₁₂ et R₁₃ pouvant être éventuellement substitués par un atome d'halogène (iode, brome, chlore, fluor) ; un groupement hydroxy (-OH); éther (-O-), amine primaire (-NH₂); amine secondaire (-NHR') ; amine tertiaire (-NR'R"), les radicaux R' et R" étant, indépendamment l'un de l'autre, un radical hydrocarboné, linéaire ou ramifié, saturé ou insaturé ayant 1 à 6 atomes de carbone, sous réserve que la somme des atomes de carbone de R₁₂ + R₁₃ + R' + R" ne dépasse pas 6. On peut citer par exemple comme monomère de ce type, le diméthylamino-éthylméthacrylamide.

Dans la formule (II), les substituants R₁₀ et X₁ sont tels que le monomère de formule (II) est hydrosoluble.

A côté des monomères hydrosolubles (la) indiqués ci-dessus toujours présents, le bloc hydrosoluble ionique A peut éventuellement être obtenu en outre à partir d'un ou plusieurs monomères hydrophobes (Ic), les dits monomères hydrophobes étant présents en une quantité suffisamment faible pour que le bloc A soit soluble dans l'eau. On peut citer par exemple comme monomères hydrophobes (Ic) utilisables dans le bloc hydrosoluble ionique A :
- le styrène et ses dérivés tel que le 4-butylstyrène, l'alpha méthylstyrène et le vinyltoluène,
- l'acétate de vinyle de formule CH₂=CH-OCOCH₃ ;
- les vinyléthers de formule CH₂=CHOR₁₄ dans laquelle R₁₄ est un radical hydrocarboné, linéaire ou ramifié, saturé ou insaturé, ayant de 1 à 6 atomes de carbone;
- l'acrylonitrile,
- la caprolactone,
- le chlorure de vinyle et le chlorure de vinylidène,
- les dérivés siliconés, conduisant après polymérisation à des polymères siliconés tels que le méthacryloxypropyltris(triméthylsiloxy)silane et les méthacrylamides siliconés,
- les monomères vinyliques hydrophobes de formule (III) suivante : 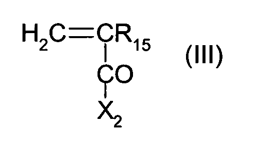
dans laquelle :
- R₁₅ est choisi parmi H, -CH₃, -C₂H₅ ou -C₃H₇
- X₂ est choisi parmi :
   - les oxydes d'alkyle de type - OR₁₆ où R₁₆ est un radical hydrocarboné, linéaire ou ramifié, saturé ou insaturé, ayant de 1 à 6 atomes de carbone.
   - les groupements -NH₂, -NHR₁₇ et -NR₁₇R₁₈ dans lesquels R₁₇ et R₁₈ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₇+ R₁₈ ne dépasse pas 6. On peut citer comme exemple de monomère de formule (III), le méthacrylate de méthyle, le méthacrylate d'éthyle, le (meth)acrylate de n-butyle, le (meth)acrylate de tertio-butyle, l'acrylate de cyclohexyle et l'acrylate d'isobornyle.

Outre les monomères hydrosolubles indiqués ci-dessus, le bloc hydrosoluble ionique A peut également être un polymère hydrosoluble ionique tel que par exemple la polyéthylène imine.

Le bloc hydrosoluble ionique A est neutralisé totalement ou partiellement par une base minérale ou organique. On entend par « partiellement neutralisé » une neutralisation d'au moins 20 % en mole. Le bloc hydrosoluble ionique A peut être neutralisé par une base minérale ou organique. La base de neutralisation peut être choisie, par exemple, parmi l'hydroxyde de sodium, les sels de sodium, ammonium, lithium, calcium, magnésium, ammonium substitué par 1 à 4 groupes alkyle portant de 1 à 15 atomes de carbone, ou encore parmi la mono-, la di-, et la triéthanolamine, l'aminoéthylpropanediol, la N-méthyl-glucamine, et les acides aminés basiques, tels que l'arginine et la lysine, et leurs mélanges.

La nature chimique et la masse molaire du bloc hydrophobe B des polymères de l'invention sont choisies de telle sorte qu'il possède une température de transition vitreuse supérieure ou égale à 70°C.

Le bloc hydrophobe B peut être obtenu à partir d'un ou plusieurs monomères hydrophobes (Id), comme par exemple :
- le styrène et ses dérivés tel que le 4-butylstyrène, l'alpha méthylstyrène et le vinyltoluène,
- l'acétate de vinyle de formule CH₂=CH-OCOCH₃ ;
- les vinyléthers de formule CH₂=CHOR₁₉ dans laquelle R₁₉ est un radical hydrocarboné, linéaire ou ramifié, saturé ou insaturé, ayant de 1 à 6 carbones;
- l'acrylonitrile,
- le chlorure de vinyle et le chlorure de vinylidène,
- la caprolactone,
- les alcènes tels que l'éthylène, le propylène, le butylène et le butadiène,
- les dérivés siliconés, conduisant après polymérisation à des polymères siliconés tels que le polydiméthylsiloxane, le méthacryloxypropyltris(triméthylsiloxy)silane et les méthacrylamides siliconés,
- les monomères vinyliques hydrophobes de formule (IV) suivante : 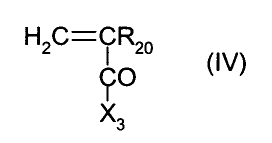
dans laquelle :
- R₂₀ est choisi parmi H, -CH₃, -C₂H₅ ou -C₃H₇;
- X₃ est choisi parmi :
   - les oxydes d'alkyle de type -OR₂₁ où R₂₁ est un radical hydrocarboné, linéaire ou ramifié, saturé ou insaturé, ayant de 1 à 22 carbones, éventuellement substitué par un atome d'halogène (iode, brome, chlore, fluor); un groupement sulfonique (-SO₃⁻), sulfate (-SO₄⁻), phosphate (-PO₄H₂⁻); hydroxy (-OH); éther (-O-), amine primaire (-NH₂); amine secondaire (-NHR₂₂) ; amine tertiaire (-NR₂₂R₂₃), ou quaternaire (-N⁺R₂₂R₂₃R₂₄), les radicaux R₂₂, R₂₃ et R₂₄ étant, indépendamment l'un de l'autre, un radical hydrocarboné, linéaire ou ramifié, saturé ou insaturé ayant 1 à 22 atomes de carbone, sous réserve que la somme des atomes de carbone de R₂₁ + R₂₂ + R₂₃ + R₂₄ ne dépasse pas 22. Comme monomères vinyliques hydrophobes comportant des groupements oxydes d'alkyle de type -OR₂₁, on peut citer par exemple le méthacrylate de méthyle, le méthacrylate d'éthyle, le (meth)acrylate de n-butyle, le (meth)acrylate de tertio-butyle, l'acrylate de cyclohexyle, l'acrylate d'isobornyle et l'acrylate d'éthyle 2-hexyle. R₂₁ peut également être un radical perfluoroalkyle, de préférence en C₁₋₁₈ : comme monomère de formule (IV) avec un radical perfluoroalkyle constituant le groupe R₂₁, on peut citer par exemple l'acrylate d'éthyle perfluorooctyle et le (méth)acrylate de trifluorométhyle.
   - les groupements -NH₂, -NHR₂₅ et -NR₂₅R₂₆ dans lesquels les radicaux R₂₅ et R₂₆ sont, indépendamment l'un de l'autre, un radical hydrocarboné, linéaire ou ramifié, saturé ou insaturé ayant 1 à 22 atomes de carbone, sous réserve que le nombre total d'atomes de carbone de R₂₅ + R₂₆ ne dépasse pas 22, lesdits radicaux R₂₅ et R₂₆ étant éventuellement substitués par un atome d'halogène (iode, brome, chlore, fluor) ; un groupement hydroxy (-OH), éther (-O-), carboxylate (-CO₂⁻), sulfonique (-SO₃⁻), sulfate (-SO4⁻), phosphate (-PO₄H₂⁻), acide carboxylique (-COOH), amine primaire (-NH₂), amine secondaire (-NHR') ; amine tertiaire (-NR'R"), ou quaternaire (-N⁺R'R"R"'), les radicaux R', R" et R"' représentant indépendamment l'un de l'autre, un radical hydrocarboné, linéaire ou ramifié, saturé ou insaturé ayant 1 à 22 atomes de carbone, sous réserve que la somme des atomes de carbone de R₂₅ + R₂₆ + R' + R" + R"' ne dépasse pas 22. R₂₅ et R₂₆ indépendamment l'un de l'autre peuvent également être un radical perfluoroalkyle, comportant de préférence de 1 à 18 atomes de carbone.

Dans la formule (IV), les substituants R₂₀ et X₃ sont tels que le monomère de formule (IV) est hydrophobe.

A côté des monomères hydrophobes (Id) indiqués ci-dessus et toujours présents dans le bloc hydrophobe B, celui-ci peut éventuellement être obtenu en outre à partir d'un ou plusieurs monomères hydrosolubles, les dits monomères hydrosolubles étant présents en une quantité suffisamment faible pour que le bloc B soit hydrophobe. Comme monomères hydrosolubles (le), on peut citer par exemple les composés suivants ou leurs sels :
- l'acide (méth)acrylique,
- l'acide styrène sulfonique,
- l'acide vinylsulfonique et l'acide (méth)allylsulfonique,
- l'acide vinyl phosphonique,
- l'acide et l'anhydride maléique;
- l'acide itaconique ;
- l'acide crotonique,
- le chlorure de diméthyldiallyl ammonium;
- le chlorure de méthylvinylimidazolium,
- le (méth)acrylamide,
- la N-vinylacétamide et la N-méthyl N-vinylacétamide,
- la N-vinylformamide et la N-méthyl N-vinylformamide,
- les N-vinyllactames comportant un groupe alkyle cyclique ayant de 4 à 9 atomes de carbone, tels que la N-vinylpyrrolidone, la N-butyrolactame et la N-vinylcaprolactame,
- l'alcool vinylique de formule CH₂=CHOH ;
- la 2-vinylpyridine et la 4-vinylpyridine,
- les monomères vinyliques hydrosolubles de formule (V) suivante : 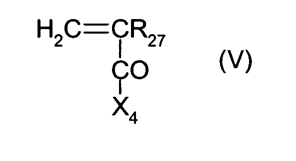
dans laquelle :
- R₂₇ est choisi parmi H, -CH₃, -C₂H₅ ou -C₃H₇;
- X₄ est choisi parmi :
   - les oxydes d'alkyle de type -OR₂₈ où R₂₈ est un radical hydrocarboné, linéaire ou ramifié, saturé ou insaturé, ayant de 1 à 6 atomes de carbone, éventuellement substitué par un atome d'halogène (iode, brome, chlore, fluor); un groupement sulfonique (-SO₃⁻), sulfate (-SO₄⁻), phosphate (-PO₄H₂⁻), hydroxy (-OH); éther (-O-), amine primaire (-NH₂); amine secondaire (-NHR') ; amine tertiaire (-NR'R"), ou quaternaire (-N⁺R'R"R"'), les radicaux R', R" et R"' étant, indépendamment l'un de l'autre, un radical hydrocarboné, linéaire ou ramifié, saturé ou insaturé ayant 1 à 6 atomes de carbone, sous réserve que la somme des atomes de carbone de R₂₈ + R' + R" + R"' ne dépasse pas 6. Comme monomères vinyliques comportant des groupes ester (X₄=OR₂₈), on peut citer par exemple le méthacrylate de diméthylaminoéthyl quaternisé (MADAME), le (méth)acrylate de glycidyle, le méthacrylate d'hydroxyéthyle, et les (méth)acrylates d'éthylène glycol, de diéthylèneglycol ou de polyalkylèneglycol.
   - les groupements -NH₂, -NHR₂₉ et -NR₂₉R₃₀ dans lesquels R₂₉ et R₃₀ sont, indépendamment l'un de l'autre, des radicaux hydrocarbonés, linéaires ou ramifiés, saturés ou insaturés ayant 1 à 6 atomes de carbone, sous réserve que le nombre total d'atomes de carbone de R₂₉ + R₃₀ ne dépasse pas 6, lesdits R₂₉ et/ou R₃₀ étant éventuellement substitués par un atome d'halogène (iode, brome, chlore, fluor); un groupement sulfonique (-SO₃⁻); sulfate (-SO₄⁻); phosphate (-PO₄H₂⁻); hydroxy (-OH); éther (-O-), amine primaire (-NH₂); amine secondaire (-NHR') ; amine tertiaire (-NR'R"), ou quaternaire (-N⁺R'R"R"'), les radicaux R', R" et R"' étant, indépendamment l'un de l'autre, un radical hydrocarboné, linéaire ou ramifié, saturé ou insaturé ayant 1 à 6 atomes de carbone, sous réserve que la somme des atomes de carbone de R₂₉ + R₃₀ + R' + R" + R"' ne dépasse pas 6. Comme monomères vinyliques hydrosolubles de formule (V) comportant des groupes amide, on peut citer par exemple l'acide 2-acrylamido-2-méthylpropane sulfonique (AMPS), le chlorure de (méth)acrylamidopropyl-triméthylammonium (APTAC et MAPTAC) et la N,N diméthylacrylamide.

Dans la formule (V), les substituants R₂₇ et X₄ sont tels que le monomère de formule (V) est hydrosoluble.

Le bloc hydrophobe B des polymères de l'invention peut être obtenu à partir de plusieurs monomères, la proportion de monomères rigides étant suffisamment importante pour que la température de transition vitreuse du bloc B soit supérieure ou égale à 70°C.

Les polymères A-B et B-A-B utilisés selon l'invention ont une masse molaire allant de 1 000 g/mole à 500 000 g/mole, de préférence de 2 000 g/mole à 100 000 g/mole. Pour les polymères diblocs, le bloc hydrosoluble ionique A a une masse molaire allant de 600 g/mole à 300 000 g/mole, de préférence de 1 200 g/mole à 60 000 g/mole, et le bloc hydrophobe B a une masse molaire allant de 400 g/mole à 200 000 g/mole, de préférence de 800 g/mole à 40 000 g/mole. Pour les polymères triblocs, le bloc hydrosoluble ionique A a une masse molaire allant de 500 g/mole à 250 000 g/mole, de préférence de 1 000 g/mole à 50 000 g/mole, et le bloc hydrophobe B a une masse molaire allant de 500 g/mole à 250 000 g/mole, de préférence de 1 000 g/mole à 50 000 g/mole.

Les polymères blocs (diblocs et triblocs) utilisés dans la composition de l'invention peuvent être préparés par les procédés de synthèse classiquement utilisés pour obtenir des polymères blocs. On peut citer par exemple comme procédé de préparation, les polymérisations de type anionique ou cationique, et la polymérisation radicalaire contrôlée qui peut être mise en oeuvre suivant différents procédés comme par exemple la voie par transfert d'atomes (Atom Transfert Radical Polymerization ou ATRP) (voir JACS, 117, page 5614 (1995), la méthode des radicaux tels que les nitroxydes (voir Georges et al., Macromolecules, 1993, 26, 2987) ou encore la voie par transfert de chaîne réversible avec addition-fragmentation (Radical Addition-Fragmentation chain Transfert) telle que le procédé MADIX (Macromolecular Design via the Interchange of Xanthate) (voir Charmot D., Corpart P., Adam H., Zard S.Z., Biadatti T., Bouhadir G., Macromol. Symp., 2000, 150, 23). Ces procédés de synthèse peuvent être utilisés pour obtenir les blocs A et B des polymères de l'invention ; ils peuvent également être utilisés pour synthétiser un seul des deux blocs du polymère de l'invention, l'autre bloc étant introduit dans le polymère final par l'intermédiaire de l'amorceur utilisé ou bien par réaction de couplage entre les blocs A et B.

La quantité de polymère(s) blocs dans la composition de l'invention peut varier selon le type de composition que l'on veut obtenir et le degré de gélification souhaitée. Elle peut aller par exemple de 0,01 à 20 % en poids, de préférence de 0,05 à 20 % en poids et mieux de 0,1 à 15 % en poids par rapport au poids total de la composition.

Les compositions cosmétiques ou dermatologiques de l'invention peuvent présenter un pH variant dans une large gamme, et pouvant aller par exemple de 2 à 10, de préférence de 3 à 8 et mieux de 4 à 7.

La phase aqueuse des compositions selon l'invention comprend au moins de l'eau. Les compositions de l'invention peuvent contenir en plus de l'eau, au moins une phase huileuse et/ou un ou plusieurs solvants organiques, hydrophiles, lipophiles et/ou amphiphiles, physiologiquement acceptables, c'est-à-dire bien tolérés et donnant un toucher cosmétiquement acceptable. La quantité de phase aqueuse peut aller par exemple de 10 à 99,99 % en poids, de préférence de 20 à 95 % en poids et mieux de 30 à 90 % en poids par rapport au poids total de la composition. L'eau peut représenter toute ou une partie de la phase aqueuse.

Les solvants organiques peuvent représenter de 5 à 50 % du poids total de la composition. Ces solvants peuvent être choisis dans le groupe constitué par les solvants organiques hydrophiles, les solvants organiques lipophiles, les solvants amphiphiles, ou leurs mélanges.

Parmi les solvants organiques, on peut citer par exemple les mono-alcools inférieurs linéaires ou ramifiés ayant de 1 à 8 atomes de carbone, comme l'éthanol, le propanol, le butanol, l'isopropanol, l'isobutanol ; les polyols tels que le propylène glycol, l'isoprène glycol, le butylène glycol, le glycérol, le sorbitol ; les mono- ou di-alkyle isosorbide dont les groupements alkyle ont de 1 à 5 atomes de carbone comme le diméthyl isosorbide ; les polyéthylène glycols notamment ceux ayant de 6 à 80 oxydes d'éthylène ; les éthers d'éthylène glycol comme le diéthylène glycol mono-méthyl ou mono-éthyl éther ; les éthers de propylène glycol comme le dipropylène glycol méthyl éther ; les esters et éthers de polyol, tels que les esters de polypropylène glycol (PPG) et plus spécialement les esters de polypropylène glycol (PPG) et d'acide gras, les éthers de PPG et d'alcool gras comme le PPG-23 oléyl éther et le PPG-36 oléate ; les esters d'acide gras et d'alkyle, tels que l'adipate de diisopropyle, l'adipate de dioctyle, les benzoates d'alkyle ; et leurs mélanges.

La composition de l'invention peut comprendre au moins une phase grasse dite aussi phase huileuse. La phase huileuse peut représenter par exemple de 0 à 80 % en poids par rapport au poids total de la composition. Dans une émulsion, la phase huileuse peut représenter par exemple de 0,1 à 50 % en poids, de préférence de 1 à 40 % en poids et mieux de 5 à 30 % en poids par rapport au poids total de la composition.

La phase grasse ou phase huileuse contient habituellement au moins une huile, notamment une huile cosmétique. Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R^{a}COOR^{b} et R^{a}OR^{b} dans laquelle R^{a} représente le reste d'un acide gras comportant de 8 à 29 atomes de carbone, et R^{b} représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12, le ceteareth-12 et le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1® " et "FLUTEC PC3® " par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060® " par la Société 3M, ou encore le bromoperfluorooctyle vendu sous la dénomination "FORALKYL® " par la Société Atochem ; le nonafluorométhoxybutane vendu sous la dénomination "MSX 4518® " par la Société 3M et le nonafluoroéthoxyisobutane ; les dérivés de perfluoromorpholine, tels que la 4-trifluorométhyl perfluoromorpholine vendue sous la dénomination "PF 5052® " par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

La composition de l'invention peut comprendre une phase aqueuse seule, ou une phase aqueuse et une phase grasse (émulsion E/H ou H/E) ou plusieurs phases aqueuses et une phase grasse (émulsion E/H/E) ou une phase aqueuse et plusieurs phases grasses (H/E/H). Elle peut ainsi constituer une solution, un gel, une émulsion (simple ou multiple).

Quand la composition est une émulsion, elle peut contenir un tensioactif émulsionnant mais selon un mode particulier de réalisation de l'invention, elle peut contenir pratiquement pas de tensioactif émulsionnant, c'est-à-dire moins de 0,5 % en poids de tensioactif émulsionnant par rapport au poids total de la composition, et même être exempte de tout tensioactif émulsionnant.

Comme exemples de tensioactifs émulsionnants pour les émulsions E/H, on peut citer par exemple les dimethicone copolyols tels que le mélange de cyclomethicone et de dimethicone copolyol, vendu sous la dénomination « DC 5225 C » par la société Dow Coming, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90^{R} par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le coémulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, on peut citer par exemple comme émulsionnants, les tensioactifs non ioniques, tels que notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, comme les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

De façon connue, les compositions de l'invention peuvent contenir des adjuvants habituels dans les domaines cosmétique et dermatologique, des gélifiants et/ou épaississants classiques hydrophiles ou lipophiles ; des polymères ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des pigments ; des charges ; des agents filmogènes ; des matières colorantes, et leurs mélanges. Les quantités de ces différents adjuvants sont celles classiquement utilisées dans les domaines considérés. Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

Comme gélifiants, on peut citer par exemple les polymères hydrophiles tels que les polymères carboxyvinyliques comme les carbomers ; les polymères d'acide 2-acrylamido-2-méthylpropane sulfonique solubles ou dispersibles en phase aqueuse comme le polymère commercialisé sous la dénomination « Hostacerin AMPS » par la société Clariant ; les polymères neutres synthétiques comme le polyvinylpyrrolidone (PVP), l'acétate de polyvinyle (PVA) ; les polysaccharides comme les gommes de guar, de xanthane et les dérivés de cellulose comme par exemple l'hydroxyéthylcellulose; les dérivés siliconés hydrosolubles ou hydrodispersibles comme les silicones acryliques et les silicones cationiques. On peut aussi utiliser des gélifiants lipophiles, tels que les argiles modifiés ou les polysaccharides modifiés.

Comme actifs utilisables dans la composition de l'invention, on peut citer par exemple les agents hydratants et par exemple les hydrolysats de protéines et les polyols tels que la glycérine, les glycols comme les polyéthylène glycols, et les dérivés de sucre ; les extraits naturels ; les agents anti-inflammatoires ; les agents apaisants ; les agents dépigmentants ; les oligomères procyannidoliques ; les vitamines comme la vitamine A (rétinol), la vitamine C (acide ascorbique), la vitamine E (tocophérol), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les protéines végétales ; les polysaccharides d'origine végétale sous forme ou non de microgels ; les dispersions de cires ; les silicates mixtes ; les enzymes ; la DHEA et ses dérivés et métabolites ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants comme les fibres ; les agents anti-chute et/ou repousse des cheveux ; les agents antirides ; les agents tenseurs tels que les polymères synthétiques ; et leurs mélanges.

Les filtres U.V. ou agents photoprotecteurs pouvant être utilisés dans la composition conforme à l'invention peut être un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US-A-4367390, EP-A-863145, EP-A-517104, EP-A-570838, EP-A-796851, EP-A-775698, EP-A-878469, EP-A-933376, EP-A-507691, EP-A-507692, EP-A-790243, EP-A-944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP-A-669323 et US-A-2,463,264; les dérivés de l'acide p-aminobenzoïque (PABA) ; les dérivés de méthylène bis-(hydroxyphényl benzotriazole) tels que décrits dans s les demandes US-A-5,237,071, US-A-5,166,355, GB-A-2303549, DE-A-197 26 184 et EP-A-893119 ; les polymères filtres et silicones filtres tels que ceux décrits notamment dans la demande WO-A-93/04665 ; les dimères dérivés d'α-alkylstyrène tels que ceux décrits dans la demande de brevet DE-A-19855649.

Comme exemples d'agents photoprotecteurs actifs dans l'UV-A et/ou l'UV-B, on peut citer désignés ci-dessous sous leur nom INCI :
- les dérivés de l'acide para-aminobenzoïique, dont les suivants : PABA, Ethyl PABA, Ethyl Dihydroxypropyl PABA, Ethylhexyl Diméthyl PABA vendu notamment sous le nom « ESCALOL 507 » par ISP, Glyceryl PABA, PEG-25 PABA vendu sous le nom « UVINUL P25 » par BASF,
- les dérivés salicyliques, dont les suivants : Homosalate vendu notamment sous le notamment sous le nom « NEO HELIOPAN OS » par HAARMANN et REIMER, Dipropyleneglycol Salicylate vendu notamment sous le nom « DIPSAL » par SCHER, TEA Salicylate vendu notamment sous le nom « NEO HELIOPAN TS » par HAARMANN et REIMER,
- les dérivés du dibenzoylméthane, dont les suivants : Butyl Methoxydibenzoylmethane vendu notamment sous le nom commercial « PARSOL 1789 » par HOFFMANN LAROCHE, Isopropyl Dibenzoylmethane,
- les dérivés cinnamiques, dont les suivants :Ethylhexyl Methoxycinnamate vendu notamment sous le nom commercial « PARSOL MCX » par HOFFMANN LAROCHE, Isopropyl Methoxy cinnamate, Isoamyl Methoxy cinnamate vendu notamment sous le nom commercial « NEO HELIOPAN E 1000 » par HAARMANN et REIMER, Cinoxate, DEA Methoxycinnamate, Diisopropyl Methylcinnamate, Glyceryl Ethylhexanoate Dimethoxycinnamate,
- les dérivés de β,β'-diphénylacrylate, dont les suivants : Octocrylene vendu notamment sous le nom commercial « UVINUL N539 » par BASF, Etocrylene vendu notamment sous le nom commercial « UVINUL N35 » par BASF,
- les dérivés de la benzophénone, dont les suivants : Benzophenone-1 vendue notamment sous le nom commercial « UVINUL 400 » par BASF, Benzophenone-2 vendue notamment sous le nom commercial « UVINUL D50 » par BASF, Benzophenone-3 ou Oxybenzone vendue notamment sous le nom commercial « UVINUL M40 » par BASF, Benzophenone-4 vendue notamment sous le nom commercial « UVINUL MS40 » par BASF, Benzophenone-5, Benzophenone-6 vendue notamment sous le nom commercial « HELISORB 11 » par NORQUAY, Benzophenone-8 vendue notamment sous le nom commercial « SPECTRA-SORB UV-24 » PAR AMERICAN CYANAMID, Benzophenone-9 vendue notamment sous le nom commercial« UVINUL DS-49» par BASF, Benzophenone-12, et le 2-(4-diéthylamino-2-hydroxybenzoyl)-benzoate de n-hexyle
- les dérivés du benzylidène camphre, dont les suivants : 3-Benzylidene camphor, 4-Methylbenzylidene camphor vendu notamment sous le nom « EUSOLEX 6300 » par MERCK , Benzylidene Camphor Sulfonic Acid, Camphor Benzalkonium Methosulfate, Terephthalylidene Dicamphor Sulfonic Acid, Polyacrylamidomethyl Benzylidene Camphor,
- les dérivés de benzimidazole, dont les suivants : Phenylbenzimidazole Sulfonic Acid vendu notamment sous le nom commercial « EUSOLEX 232 » par MERCK, Disodium Phenyl Dibenzimidazole Tetra-sulfonate vendu notamment sous le nom commercial commercial « NEO HELIOPAN AP » par HAARMANN et REIMER,
- les dérivés de triazine, dont les suivants : Anisotriazine vendu notamment sous le nom commercial «TINOSORB S » par CIBA SPECIALTY CHEMICALS, Ethylhexyl triazone vendu notamment sous le nom commercial «UVINUL T150 » par BASF, Diethylhexyl Butamido Triazone vendnotamment sous le nom commercial « UVASORB HEB » par SIGMA 3V, et la 2,4,6- tris-(4'amino-benzalmalonate de diisobutyle)-s-triazine,
- les dérivés de benzotriazole, dont les suivants : Drometrizole Trisiloxane vendu sous le nom « SILATRIZOLE » par RHODIA CHIMIE, Methylène bis-Benzotriazolyl Tetramethylbutylphénol, vendu notamment sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,
- les dérivés anthranilique, dont le Menthyl anthranilate vendu sous le nom commercial commercial « NEO HELIOPAN MA » par HAARMANN et REIMER,
- les dérivés d'imidazolines, dont l'Ethylhexyl Dimethoxybenzylidene Dioxoimidazoline Propionate,
- les dérivés de benzalmalonate, dont le polyorganosiloxane à fonctions benzalmalonate vendu sous la dénomination commerciale « PARSOL SLX » par HOFFMANN LAROCHE,
- et leurs mélanges.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, et leurs mélanges.

Les agents photoprotecteurs inorganiques peuvent être choisis parmi des pigments ou bien encore des nanopigments (taille moyenne des particules primaires: généralement entre 5 nm et 100 nm, de préférence entre 10 nm et 50 nm) d'oxydes métalliques, enrobés ou non enrobés, comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-518772 et EP-A-518773. On peut citer par exemple le nanotitane hydrophile commercialisé sous la dénomination MIRASUN TIW60 par la société Rhodia, et le nanotitane lipophile commercialisé sous la dénomination MT100T par la société TAYCA ou UV Titan M 160 par la société KEMIRA.

Les filtres U.V. sont généralement présents dans les compositions selon l'invention dans des proportions allant de 0,1 à 20% en poids par rapport au poids total de la composition, et de préférence allant de 0,2 à 15% en poids par rapport au poids total de la composition.

La composition peut, sans être déstabilisée, contenir des particules solides, comme par exemple, sans que cette liste soit exhaustive, les filtres physiques tels que ceux indiqués ci-dessus, les pigments, les charges et leurs mélanges.

Comme pigments, on peut citer les pigments minéraux et notamment les oxydes métalliques tels que les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré ; et les pigments organiques tels que le noir de carbone et les laques qui sont des sels de calcium, de baryum, d'aluminium ou de zirconium, de colorants acides tels que les colorants halogéno-acides, azoïques ou anthraquinoniques.

Ces pigments peuvent être traités de manière à rendre leur surface hydrophobe; ce traitement peut être effectué selon les méthodes connues de l'homme du métier ; les pigments peuvent notamment être enrobés par des composés siliconés tels que des PDMS et/ou par des polymères.

Comme charges, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; les particules colloïdales de charges inorganiques telles que les nanoparticules de silices colloïdales comme le produit commercialisé sous la dénomination Cosmo S40 par la société Catalysts and Chemicals et le produit commercialisé sous la dénomination Ludox AM par la société Grace ; et leurs mélanges.

Les compositions selon l'invention peuvent se présenter sous forme de gels, de lotions, de laits, de crèmes plus ou moins onctueuses, de pâtes. Ces compositions sont préparées selon les méthodes usuelles. Le polymère bloc utilisé étant hydrosoluble, il est généralement introduit dans une phase aqueuse.

Les compositions de l'invention peuvent être utilisées comme produit de soin, de traitement, de protection, de nettoyage, de démaquillage, et /ou de maquillage des matières kératiniques (peau, cheveux, cuir chevelu, cils, sourcils, ongles ou muqueuses) tels que des crèmes de protection, de traitement ou de soin pour le visage, pour les mains ou pour le corps, des laits corporels de protection ou de soin, des gels ou mousses pour le soin de la peau et/ou des muqueuses (lèvres).

Les compositions de l'invention peuvent contenir des filtres solaires et être ainsi également utilisées comme produit de protection solaire.

Les compositions peuvent être utilisées comme produits pour le maquillage, notamment le maquillage de la peau, des sourcils, des cils et des lèvres, tels que des crèmes pour le visage, des fonds de teint, des mascaras, des rouges à lèvres. De tels produits contiennent généralement des pigments.

Les compositions selon l'invention peuvent être également utilisées comme produits rincés ou comme produits non-rincés pour le nettoyage de la peau du visage et/ou du corps et/ou pour le nettoyage des cheveux, par exemple comme produits capillaires y compris pour le soin et le conditionnement des cheveux.

L'invention a pour objet l'utilisation cosmétique d'une composition cosmétique telle que définie ci-dessus, comme produit capillaire rincé ou non-rincé.

L'invention a pour objet l'utilisation cosmétique d'une composition cosmétique telle que définie ci-dessus, comme produit de nettoyage et/ou de démaquillage de la peau et/ou des yeux.

L'invention a aussi pour objet l'utilisation cosmétique d'une composition cosmétique telle que définie ci-dessus, comme produit de soin pour la peau, les cheveux, le cuir chevelu, les cils, les sourcils, les ongles ou les muqueuses.

L'invention a aussi pour objet l'utilisation cosmétique d'une composition cosmétique telle que définie ci-dessus, comme produit de maquillage.

L'invention a aussi pour objet l'utilisation cosmétique d'une composition cosmétique telle que définie ci-dessus, comme produit de protection solaire (protection contre le soleil et/ou les U.V. des appareils à bronzer).

Un autre objet de l'invention est un procédé de traitement cosmétique (non-thérapeutique) d'une matière kératinique telle que la peau, le cuir chevelu, les cheveux, les cils, les sourcils, les ongles ou les muqueuses, caractérisé en ce qu'on applique sur la matière kératinique, une composition cosmétique telle que définie ci-dessus.

### Exemples

Les exemples qui suivent servent à illustrer l'invention sans toutefois présenter un caractère limitatif. Les quantités indiquées sont en % en poids sauf mention contraire.

### Exemple 1 : Dispersions aqueuses de nano-oxyde de titane (5%) comprenant 2% (en poids) d'un aélifiant hydrophile.

Le pouvoir gélifiant des polymères a été mis en évidence par rhéologie à l'aide d'un rhéomètre de type RS150 (Haake) fonctionnant à contrainte imposée, et équipé d'une géométrie cône/plan 35mm/2°. Un système de régulation en température à effet Peltier permet d'assurer une mise en température de l'échantillon à 20°C pendant les mesures. Les caractérisations rhéologiques ont été réalisées en modes écoulement et dynamique.

### Mesures en écoulement:

Les mesures sont effectuées en imposant une rampe ascendante et une rampe descendante en contrainte à l'équilibre de 0 Pa jusqu'à une contrainte correspondant à une vitesse de cisaillement de 500 s⁻¹. Ces mesures permettent d'évaluer la viscosité des systèmes étudiés pour des vitesses de cisaillement égales à 0,01 s⁻¹ et 100 s⁻¹.

### Mesures en dynamique: 10⁻²

Les limites des domaines viscoélastiques linéaires ont été déterminées à 10⁻² et 1 Hz en soumettant l'échantillon à une série de contraintes sinusoïdales de fréquence fixe et d'amplitudes croissantes réparties de façon logarithmique entre deux bornes à raison de 5 points par décade. Le comportement viscoélastique linéaire est ensuite caractérisé en soumettant l'échantillon à une série de 20 contraintes sinusoïdales de fréquences logarithmiquement réparties entre 10 et 10⁻² Hz et d'amplitudes telles que l'amplitude de déformation soit constante et située dans le domaine linéaire précédemment déterminé. Ces mesures permettent d'évaluer le module complexe G* à 1 Hz et l'angle de perte δ à 1 Hz, dans le domaine de viscoélasticité linéaire. G* et δ sont les paramètres viscoélastiques utilisés pour mesurer les propriétés physiques des fluides viscoélastiques, comme expliqué dans « An introduction to rheology » de H.A BARNES, J.F HUTTON, K. WALTERS, pages 46 à 54 (Ed. Elsevier 1989). Le module G* est égale à la racine carré de la somme des carrés du module élastique G' (module de conservation ou « storage modulus ») et du module de perte G" (module de perte ou « loss modulus »'). La tangente de l'angle de perte δ est égale au rapport G"/G'.

### 1) Gels aqueux :

Les trois gélifiants suivants ont été utilisés dans les dispersions données en exemple :
Gélifiant 1 (selon l'invention) : Copolymère diblocs polystyrène 4300 g/mole - b-polyacrylate de sodium 25460 g/mole (b=bloc). La température de transition vitreuse du bloc polystyrène est égale à 76°C.
Gélifiant 2 (art antérieur selon le document EP-A-1,279,398) : Copolymère diblocs polystyrène 1800 g/mole - b - polyacrylate de sodium 42430 g/mole. La température de transition vitreuse du bloc polystyrène est égale à 45°C.
Gélifiant 3 (art antérieur) : Polyacrylate de sodium réticulé (Carbopol 980 fourni par la société Novéon)

*Préparation des gels:* Les gels aqueux comprenant 2% (en poids) des gélifiants 1 et 2 sont préparés par dissolution de la quantité adéquate de polymère sous forme poudre dans l'eau, sous agitation à 90°C pendant 5 heures, dans un flacon fermé hermétiquement. Le pH est ajusté à 7 par ajout de la quantité adéquate de soude 2 mole/l. Les gels obtenus sont transparents.

Le gel aqueux comprenant 2% (en poids) du gélifiant 3 est préparé par dissolution de la quantité adéquate de polymère sous forme poudre dans l'eau, sous agitation à 25°C pendant 2 heures. Le pH est ajusté à 7 par ajout de la quantité adéquate d'hydroxyde de sodium à 2 mole/l. Le gel obtenu est transparent.

### Caractéristiques rhéologiques des gels aqueux

Les trois gels aqueux comprenant 2 % en poids de polymère ont été caractérisés suivant le protocole indiqué ci-dessus. Ces gels possèdent les caractéristiques rhéologiques suivantes :

| | Viscosité (0,01 s⁻¹) (Pa.s) | Viscosité (100 s⁻¹) (Pa.s) | G*(1 Hz) (Pa) | δ (1 Hz) (°) |
|---|---|---|---|---|
| Gélifiant 1 | 8 000 | 10 | 6 000 | 2 |
| Gélifiant 2 | 7 000 | 4 | 600 | 5 |
| Gélifiant 3 | 4 000 | 5 | 700 | 8 |

Le gélifiant 1 présente à 2 %, un pouvoir gélifiant de l'eau supérieur à celui des gélifiants 2 et 3, notamment pour des sollicitations de faible amplitude (Module complexe élevé et faible angle de perte).

### 2) Dispersions aqueuses gélifiées de nano-oxyde de titane à 5 % en poids

Les trois gels aqueux décrits précédemment ont été utilisés pour obtenir des dispersions de nano-oxyde de titane (TiO₂) comprenant 5 % en poids de TiO₂ par rapport au poids total du gel.

*Préparation des dispersions :* Les particules de TiO₂ sont ajoutées aux gels aqueux décrits ci dessus, à 25°C, sous agitation à l'aide d'un homogénéisateur de type Ultraturax pendant 5 minutes à la vitesse de 24 000 RPM. L'axe utilisé a un diamètre extérieur de 10 mm (référence 10F).

### Stabilités des dispersions de TiO₂

Les dispersions comprenant 5 % en poids de TiO₂ et 2 % en poids de gélifiant par rapport au poids total du gel, ont été soumises à un test de vieillissement accéléré par centrifugation pendant 5 minutes à la vitesse de 28 000 RPM à l'aide d'une centrifugeuse de type Beckman Optima TLX équipée d'un rotor de type TLA 100.4. 0,5 gramme de dispersion sont prélevés en haut et en bas des tubes de centrifugation ; leur extrait sec est alors évalué à l'aide d'une balance Sartorius MA 100 (mesure de la masse résiduelle à 105°C).

| | | Extrait sec (%) |
|---|---|---|
| Dispersion comprenant le gélifiant 1 | Haut du tube | 7,0 |
| | Bas du tube | 7,4 |
| Dispersion comprenant le gélifiant 2 | Haut du tube | 3,2 |
| | Bas du tube | 30,6 |
| Dispersion comprenant le gélifiant 3 | Haut du tube | 5,9 |
| | Bas du tube | 21,8 |

Les résultats montrent que la dispersion de TiO₂ comprenant 2 % du gélifiant 1 utilisé selon l'invention reste stable après centrifugation et qu'il ne se produit pas de sédimentation, contrairement aux dispersions comprenant les gélifiants 2 et 3 dans lesquelles les particules inorganiques ont sédimentées (différences des proportions d'extrait sec entre le haut et le bas du tube). Ainsi, le gélifiant 1 selon l'invention permet d'obtenir une dispersion aqueuse de TiO₂ plus stable que celles comprenant les gélifiants 2 et 3.

### Exemple 2 selon l'invention et exemple comparatif 3 : crèmes hydratantes

| Composition | Exemple 2 selon l'invention | Exemple 3 comparatif |
|---|---|---|
| *Phase huileuse :* | | |
| Huile de Parléam | 20 % | 20 % |
| | | |

| *Phase aqueuse :* | | |
|---|---|---|
| Glycérine | 3% | 3% |
| Copolymère diblocs polystyrène 4300 g/mole - b - polyacrylate de sodium 25460 g/mole | 1 % | 0% |
| Polyacrylate de sodium réticulé (Carbopol 980 fourni par la société Novéon) | 0% | 1% |
| Triéthanolamine | 0% | 0,05% |
| Eau déminéralisée | Qsp 100% | Qsp 100% |

Mode de préparation des émulsions H/E : Les polymères sont dissous dans la phase aqueuse par simple agitation pendant 5 heures à 90°C pour le polymère bloc et pendant 2 heures à 25°C pour le polyacrylate de sodium. La phase huileuse est alors introduite progressivement dans la phase aqueuse à la température ambiante sous agitation à l'aide d'un homogénéisateur de type Ultraturax pendant 5 minutes à la vitesse de 24 000 RPM (référence 10F).

*Caractéristiques des émulsions:* L'émulsion de l'exemple 2 selon l'invention est une crème blanche et homogène dont la taille moyenne des gouttes d'huile est égale à 10 microns. L'émulsion de l'exemple 3 comparatif est macroscopiquement hétérogène dès sa préparation avec formation d'un film d'huile en surface, correspondant à 30 % de la phase huileuse qui, donc, n'a pas été émulsionnée.

Ainsi, le polymère diblocs utilisé selon l'invention permet de stabiliser une émulsion sans tensioactif comprenant 20% de phase huileuse, contrairement au polyacrylate de sodium réticulé utilisé dans l'art antérieur.

### Exemple 4 selon l'invention :

On peut préparer une crème de manière analogue à l'exemple 2, en utilisant au lieu du polymère diblocs, le polymère triblocs suivant : Polystyrène 4300 g/mole / Polyacrylate de sodium 49000 g/mole / Polystyrène 4300 g/mole. La proportion massique du bloc hydrophile est égale à 85%.

## Revendications

1. Composition pour application topique, **caractérisée en ce qu'**elle comprend au moins une phase aqueuse comportant au moins un polymère hydrosoluble ou hydrodispersible, choisi parmi (1) les polymères de structure triblocs B-A-B où A est un bloc polymérique hydrosoluble ionique et B un bloc polymérique hydrophobe, où la quantité de bloc polymérique A est égale ou supérieure à 50 % du poids total du polymère triblocs B-A-B, et où le bloc B hydrophobe a une température de transition vitreuse supérieure ou égale à 70°C, et/ou (2) les polymères de structure diblocs A-B, où A est un bloc polymérique hydrosoluble ionique et B un bloc polymérique hydrophobe, où la quantité de bloc polymérique A est égale ou supérieure à 60 % du poids total du polymère diblocs A-B et où le bloc B hydrophobe a une température de transition vitreuse supérieure ou égale à 70°C.

2. Composition selon la revendication 1, **caractérisée en ce que** le bloc hydrosoluble ionique A est obtenu à partir d'un ou plusieurs monomères hydrosolubles (la), ou de leurs sels, les monomères (la) étant choisis parmi :
- l'acide (méth)acrylique,
- l'acide styrène sulfonique,
- l'acide vinylsulfonique et l'acide (méth)allylsulfonique,
- l'acide vinyl phosphonique,
- l'acide maléique,
- l'acide itaconique,
- l'acide crotonique,
- le chlorure de diméthyldiallyl ammonium,
- le chlorure de méthylvinylimidazolium,
- les carboxybétaïnes ou sulfobétaïnes éthyléniques,
- les monomères vinyliques hydrosolubles de formule (I) suivante : 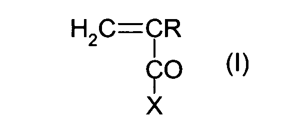
dans laquelle :
- R est choisi parmi H, -CH₃, -C₂H₅ ou -C₃H₇ ;
- X est choisi parmi :
- les oxydes d'alkyle de type -OR₁ où R₁ est un radical hydrocarboné, linéaire ou ramifié, saturé ou insaturé, ayant de 1 à 6 atomes de carbone, substitué par au moins un groupement carboxylate, sulfonique, sulfate, phosphate et/ou ammonium quaternaire (-N⁺R₂R₃R₄), les radicaux R₂, R₃ et R₄ étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R₂ + R₃ + R₄ ne dépasse pas 6 ; le radical R₁ étant éventuellement substitué par un atome d'halogène ; un groupement hydroxy ; acide carboxylique ; éther; amine primaire ; amine secondaire (-NHR') ; amine tertiaire (-NR'R"), les radicaux R' et R" étant, indépendamment l'un de l'autre, un radical hydrocarboné, linéaire ou ramifié, saturé ou insaturé ayant 1 à 6 atomes de carbone, sous réserve que la somme des atomes de carbone de R₁ + R' + R" ne dépasse pas 6 ;
- les groupements -NH₂, - NHR₅ et -NR₅R₆ dans lesquels R₅ et R₆ sont, indépendamment l'un de l'autre, un radical hydrocarboné, linéaire ou ramifié, saturé ou insaturé ayant 1 à 6 atomes de carbone, sous réserve que le nombre total d'atomes de carbone de R₅ + R₆ ne dépasse pas 6, lesdits radicaux R₅ et/ou R₆ étant substitués par au moins un groupement carboxylate, sulfonique, sulfate, phosphate et/ou amine quaternaire (-N⁺R₇R₈R₉), les radicaux R₇, R₈ et R₉ étant, indépendamment l'un de l'autre, un radical hydrocarboné, linéaire ou ramifié, saturé ou insaturé ayant 1 à 6 atomes de carbone, sous réserve que la somme des atomes de carbone de R₅ + R₆ + R₇ + R₈ + R₉ ne dépasse pas 6 ; les radicaux R₅ et/ou R₆ étant éventuellement substitués par un atome d'halogène ; ou un groupement hydroxy ; éther ; acide carboxylique ; amine primaire ; amine secondaire (-NHR') ; amine tertiaire (-NR'R"), les radicaux R" et R", étant, indépendamment l'un de l'autre, un radical hydrocarboné, linéaire ou ramifié, saturé ou insaturé ayant 1 à 6 atomes de carbone, sous réserve que la somme des atomes de carbone de R₇ + R₈ + R' + R" ne dépasse pas 6.

3. Composition selon la revendication 1 ou 2, **caractérisée en ce que** le bloc hydrosoluble ionique A est obtenu en outre à partir d'un ou plusieurs monomères choisis parmi les monomères hydrosolubles neutres (Ib), les monomères hydrophobes (Ic), et leurs mélanges.

4. Composition selon la revendication 3, **caractérisée en ce que** le monomère hydrosoluble neutre (Ib) est choisi parmi :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyle cyclique ayant de 4 à 9 atomes de carbone,
- l'alcool vinylique,
- les monomères vinyliques hydrosolubles de formule (II) suivante : 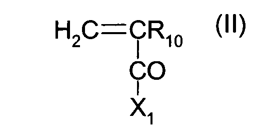
dans laquelle :
- R₁₀ est choisi parmi H, -CH₃, -C₂H₅ ou -C₃H₇
- X₁ est choisi parmi :
- les oxydes d'alkyle de type -OR₁₁ où R₁₁ est un radical hydrocarboné, linéaire ou ramifié, saturé ou insaturé, ayant de 1 à 6 carbones, éventuellement substitué par un atome d'halogène (iode, brome, chlore, fluor) ; un groupement hydroxy (-OH); éther (-O-), amine primaire (-NH₂); amine secondaire (-NHR') ; amine tertiaire (-NR'R"), les radicaux R' et R" étant, indépendamment l'un de l'autre, un radical hydrocarboné, linéaire ou ramifié, saturé ou insaturé ayant 1 à 6 atomes de carbone, sous réserve que la somme des atomes de carbone de R₁₁ + R' + R" ne dépasse pas 6.
- les groupements -NH₂, -NHR₁₂ et -NR₁₂R₁₃ dans lesquels R₁₂ et R₁₃ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₂ et R₁₃ ne dépasse pas 6, les radicaux R₁₂ et R₁₃ pouvant être éventuellement substitués par un atome d'halogène (iode, brome, chlore, fluor) ; un groupement hydroxy (-OH); éther (-O-), amine primaire (-NH₂); amine secondaire (-NHR') ; amine tertiaire (-NR'R"), les radicaux R' et R" étant, indépendamment l'un de l'autre, un radical hydrocarboné, linéaire ou ramifié, saturé ou insaturé ayant 1 à 6 atomes de carbone, sous réserve que la somme des atomes de carbone de R₁₂ + R₁₃ + R' + R" ne dépasse pas 6.

5. Composition selon la revendication 3, **caractérisée en ce que** le monomère hydrophobe (Ic) est choisi parmi :
- le styrène et ses dérivés,
- l'acétate de vinyle,
- les vinyléthers de formule CH₂=CHOR₁₄ dans laquelle R₁₄ est un radical hydrocarboné, linéaire ou ramifié, saturé ou insaturé, ayant de 1 à 6 atomes de carbone ;
- l'acrylonitrile ;
- la caprolactone ;
- le chlorure de vinyle et le chlorure de vinylidène ;
- les monomères siliconés insaturés ;
- les monomères vinyliques hydrophobes de formule (III) suivante : 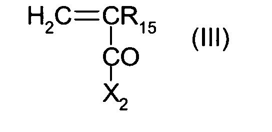
dans laquelle :
- R₁₅ est choisi parmi H, -CH₃, -C₂H₅ ou -C₃H₇
- X₂ est choisi parmi :
- les oxydes d'alkyle de type - OR₁₆ où R₁₆ est un radical hydrocarboné, linéaire ou ramifié, saturé ou insaturé, ayant de 1 à 6 atomes de carbone ;
- les groupements -NH₂, -NHR₁₇ et -NR₁₇R₁₈ dans lesquels R₁₇ et R₁₈ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₁₇+ R₁₈ ne dépasse pas 6.

6. Composition selon la revendication 1, **caractérisée en ce que** le bloc hydrosoluble ionique A est un polymère hydrosoluble ionique.

7. Composition selon la revendication précédente, **caractérisée en ce que** le bloc hydrosoluble ionique A est la polyéthylène imine.

8. Composition selon l'une quelconque des revendications précédentes, **caractérisée en ce que** le bloc hydrosoluble ionique A est totalement ou partiellement neutralisé par une base minérale ou organique.

9. Composition selon l'une quelconque des revendications précédentes, **caractérisée en ce que** le bloc hydrophobe B est obtenu à partir d'un ou plusieurs monomères hydrophobes (Id), choisis parmi :
- le styrène et ses dérivés,
- l'acétate de vinyle,
- les vinyléthers de formule CH₂=CHOR₁₉ dans laquelle R₁₉ est un radical hydrocarboné, linéaire ou ramifié, saturé ou insaturé, ayant de 1 à 22 atomes de carbone,
- l'acrylonitrile,
- le chlorure de vinyle et le chlorure de vinylidène,
- la caprolactone,
- les alcènes,
- les dérivés siliconés, conduisant après polymérisation à des polymères siliconés,
- les monomères vinyliques hydrophobes de formule (IV) suivante : 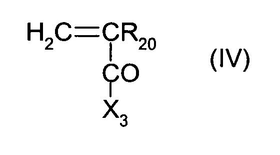
dans laquelle :
- R₂₀ est choisi parmi H, -CH₃, -C₂H₅ ou -C₃H₇ ;
- X₃ est choisi parmi :
- les oxydes d'alkyle de type -OR₂₁ où R₂₁ est un radical hydrocarboné, linéaire ou ramifié, saturé ou insaturé, ayant de 1 à 22 carbones, éventuellement substitué par un atome d'halogène (iode, brome, chlore, fluor); un groupement sulfonique (-SO₃⁻), sulfate (-SO₄⁻), phosphate (-PO₄H₂⁻); hydroxy (-OH); éther (-O-), amine primaire (-NH₂); amine secondaire (-NHR₂₂) ; amine tertiaire (-NR₂₂R₂₃), ou quaternaire (-N⁺R₂₂R₂₃R₂₄), les radicaux R₂₂, R₂₃ et R₂₄ étant, indépendamment l'un de l'autre, un radical hydrocarboné, linéaire ou ramifié, saturé ou insaturé ayant 1 à 22 atomes de carbone, sous réserve que la somme des atomes de carbone de R₂₁ + R₂₂ + R₂₃ + R₂₄ ne dépasse pas 22 ; R₂₁ pouvant également être un radical perfluoroalkyle.
- les groupements -NH₂, -NHR₂₅ et -NR₂₅R₂₆ dans lesquels les radicaux R₂₅ et R₂₆ sont, indépendamment l'un de l'autre, un radical hydrocarboné, linéaire ou ramifié, saturé ou insaturé ayant 1 à 22 atomes de carbone, sous réserve que le nombre total d'atomes de carbone de R₂₅ + R₂₆ ne dépasse pas 22, lesdits radicaux R₂₅ et R₂₆ étant éventuellement substitués par un atome d'halogène (iode, brome, chlore, fluor) ; un groupement hydroxy (-OH), éther (-O-), carboxylate (-CO₂⁻), sulfonique (-SO₃⁻), sulfate (-SO₄⁻), phosphate (-PO₄H₂⁻), acide carboxylique (-COOH), amine primaire (-NH₂), amine secondaire secondaire (-NHR') ; amine tertiaire (-NR'R"), ou quaternaire (-N⁺R'R"R"'), les radicaux R', R" et R"' représentant indépendamment l'un de l'autre, un radical hydrocarboné, linéaire ou ramifié, saturé ou insaturé ayant 1 à 22 atomes de carbone, sous réserve que la somme des atomes de carbone de R₂₅ + R₂₆ + R' + R" + R"' ne dépasse pas 22 ; R₂₅ et R₂₆ indépendamment l'un de l'autre pouvant également être un radical perfluoroalkyle.

10. Composition selon la revendication précédente, **caractérisée en ce que** le bloc hydrophobe B est obtenu en outre à partir d'un ou plusieurs monomères hydrosolubles ioniques ou neutres (le) choisis parmi les monomères suivants, ou leurs sels :
- l'acide (méth)acrylique ;
- l'acide styrène sulfonique ;
- l'acide vinylsulfonique et l'acide (méth)allylsulfonique ;
- l'acide vinyl phosphonique ;
- l'anhydride maléique ;
- l'acide maléique ;
- l'acide itaconique ;
- l'acide crotonique ;
- le chlorure de diméthyldiallyl ammonium ;
- le chlorure de méthylvinylimidazolium ;
- le (méth)acrylamide ;
- la N-vinylacétamide et la N-méthyl N-vinylacétamide ;
- la N-vinylformamide et la N-méthyl N-vinylformamide ;
- les N-vinyllactames comportant un groupe alkyle cyclique ayant de 4 à 9 atomes de carbone ;
- l'alcool vinylique ;
- la 2-vinylpyridine et la 4-vinylpyridine ;
- les monomères vinyliques hydrosolubles de formule (V) suivante : 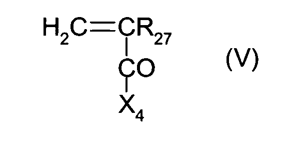
dans laquelle :
- R₂₇ est choisi parmi H, -CH₃, -C₂H₅ ou -C₃H₇ ;
- X₄ est choisi parmi :
- les oxydes d'alkyle de type -OR₂₈ où R₂₈ est un radical hydrocarboné, linéaire ou ramifié, saturé ou insaturé, ayant de 1 à 6 atomes de carbone, éventuellement substitué par un atome d'halogène (iode, brome, chlore, fluor); un groupement sulfonique (-SO₃⁻), sulfate (-SO₄⁻), phosphate (-PO₄H₂⁻), hydroxy (-OH); éther (-O-), amine primaire (-NH₂); amine secondaire (-NHR') ; amine tertiaire (-NR'R"), ou quaternaire (-N⁺R'R"R"'), les radicaux R', R" et R"' étant, indépendamment l'un de l'autre, un radical hydrocarboné, linéaire ou ramifié, saturé ou insaturé ayant 1 à 6 atomes de carbone, sous réserve que la somme des atomes de carbone de R₂₈ + R' + R" + R"' ne dépasse pas 6.
- les groupements -NH₂, -NHR₂₉ et -NR₂₉R₃₀ dans lesquels R₂₉ et R₃₀ sont, indépendamment l'un de l'autre, des radicaux hydrocarbonés, linéaires ou ramifiés, saturés ou insaturés ayant 1 à 6 atomes de carbone, sous réserve que le nombre total d'atomes de carbone de R₂₉ +t R₃₀ ne dépasse pas 6, lesdits R₂₉ et/ou R₃₀ étant éventuellement substitués par un atome d'halogène (iode, brome, chlore, fluor); un groupement sulfonique (-SO₃⁻); sulfate (-SO₄⁻ ); phosphate (-PO₄H₂⁻); hydroxy (-OH); éther (-O-), amine primaire (-NH₂); amine secondaire (-NHR') ; amine tertiaire (-NR'R"), ou quaternaire (-N⁺R'R"R"'), les radicaux R', R" et R"' étant, indépendamment l'un de l'autre, un radical hydrocarboné, linéaire ou ramifié, saturé ou insaturé ayant 1 à 6 atomes de carbone, sous réserve que la somme des atomes de carbone de R₂₉ + R₃₀ + R' + R" + R"' ne dépasse pas 6.

11. Composition selon l'une quelconque des revendications précédentes, **caractérisée en ce que** le polymère a une masse molaire allant de 1 000 g/mole à 500 000 g/mole.

12. Composition selon l'une quelconque des revendications précédentes, **caractérisée en ce que** la proportion du bloc hydrophile ionique A dans le polymère diblocs est supérieure à 70 % en poids par rapport au poids total des blocs A et B.

13. Composition selon l'une quelconque des revendications précédentes, **caractérisée en ce que** la proportion du bloc hydrophile ionique A dans le polymère triblocs est supérieure à 60 % en poids par rapport au poids total des blocs A et B.

14. Composition selon la revendication 1, **caractérisée en ce que** le polymère comporte, comme bloc A, du polyacrylate de sodium et, comme bloc B, du polystyrène.

15. Composition selon l'une quelconque des revendications précédentes, **caractérisée en ce que** la quantité de polymère(s) va de 0,01 à 20 % en poids par rapport au poids total de la composition.

16. Composition selon l'une quelconque des revendications précédentes, **caractérisée en ce qu'**elle comprend au moins une phase huileuse.

17. Composition selon la revendication précédente, **caractérisée en ce qu'**elle contient des particules solides choisies parmi les filtres physiques, les pigments, les charges et leurs mélanges.

18. Composition selon l'une quelconque des revendications précédentes, **caractérisée en ce qu'**elle constitue une composition cosmétique ou dermatologique.

19. Composition selon l'une quelconque des revendications précédentes, **caractérisée en ce qu'**elle constitue un produit de soin, de traitement, de protection, de nettoyage, de démaquillage et /ou de maquillage des matières kératiniques, notamment la peau.

20. Utilisation cosmétique d'une composition cosmétique selon l'une quelconque des revendications 1 à 17, comme produit de soin pour la peau, les cheveux, le cuir chevelu, des cils, des sourcils, des ongles ou des muqueuses, comme produit de maquillage, comme produit de protection solaire, comme produit capillaire rincé ou non-rincé, ou comme produit de nettoyage et/ou de démaquillage de la peau et/ou des yeux.

21. Procédé de traitement cosmétique d'une matière kératinique, **caractérisé en ce que** l'on applique sur la matière kératinique, une composition cosmétique telle que définie selon l'une quelconque des revendications 1 à 17.

22. Utilisation d'au moins un polymère hydrosoluble ou hydrodispersible, choisi parmi (1) les polymères de structure triblocs B-A-B où A est un bloc polymérique hydrosoluble ionique et B un bloc polymérique hydrophobe, où la quantité de bloc polymérique A est égale ou supérieure à 50 % du poids total du polymère triblocs B-A-B, et où le bloc B hydrophobe a une température de transition vitreuse supérieure ou égale à 70°C, et/ou (2) les polymères de structure diblocs A-B, où A est un bloc polymérique hydrosoluble ionique et B un bloc polymérique hydrophobe, où la quantité de bloc polymérique A est égale ou supérieure à 60 % du poids total du polymère diblocs A-B et où le bloc B hydrophobe a une température de transition vitreuse supérieure ou égale à 70°C, pour stabiliser une composition cosmétique et/ou dermatologique comprenant au moins une phase aqueuse et des particules solides.
